# EUROPEAN PATENT SPECIFICATION

(11) **EP 0 462 082 B1**
(45) Date of publication and mention of the grant of the patent: **02.04.1997**
(21) Application number: 91830258.9
(22) Date of filing: 11.06.1991
(51) Int. Cl.: C12N 11/14, C02F 11/02

(54) **Method of binding biological catalytic activities to sintered expanded clays and product obtained therefrom**
Verfahren zur Bindung biologischer katalytischer Aktivitäten an gesinterte expandierte Tone und dadurch erhaltene Produkte
Méthode de couplage entre des activités catalytiques biologiques et des argiles expansées et frittées et produits ainsi obtenus

(30) Priority: 13.06.1990 IT 4805890
(43) Date of publication of application: 18.12.1991
(73) Proprietor: ENTE PER LE NUOVE TECNOLOGIE, L'ENERGIA E L'AMBIENTE ( ENEA), I-00198 Roma RM (IT); CONSIGLIO NAZIONALE DELLE RICERCHE, 00185 Roma (IT)
(72) Inventor: Capuano, Vincenzo, I-00061 Anguillara Sabazia Roma (IT); Cervelli, Stefano, I-56100 Pisa (IT)
(74) Representative: Sarpi, Maurizio

(56) References cited:
- EP-A- 0 271 289
- DE-A- 2 633 259
- US-B- 395 975

## Description

The present invention relates to a method for binding biological catalytic activities (enzymes, cells cellular organelles) to substrates formed by sintered expanded clays as well as the products obtained from said method which can be used in agriculture as catalysts of processes adapted to change and improve the efficiency of not very fertile lands, in the decontamination operations, in the city and industrial waste dumps, in the industrial processes for depurating mud and waste water, and generally in any field where transformation reactions should be catalyzed.

Transformation of agricultural, industrial, and city wastes is one of the most delicate problems to be solved at present. Hitherto made attempts have been aiming at the solution of particular cases. Moreover the biotechnology applied to this field has not given any expected result so far because, even if genetically modified microorganisms having the capability of degrading the poison and yet harmful waste materials have been developed, the use of such microorganisms in agriculture or in decontamination operations is subjected to the release thereof into the environment, which is causing many problems also due to the risk of transferring the modified genetic characteristics to other organisms.

In contrast, there is provided according to the present invention the use of one or more enzymes bound on materials which are not expensive, becomes integrated in the application environment, and do not affect but positively modify the catalytic activities by protecting them with the time and in the operation fields. The use of such bound biological catalytic activities, even if they are obtained from genetically modified microorganisms, does not imply the above mentioned problems nor meet with application difficulties.

Several techniques of binding enzymes and cells have been known for many years, such techniques being aimed at the binding or localization of such enzymes during continuous catalytic processes.

The purpose of such binding techniques has been achieved either by covalent bonding with insoluble or made insoluble "functionalized" polymers, or by the absorption on organic or inorganic insoluble substrates, or by the entrapping within gelatinous matrices or semipermeable microcapsules.

The essential reasons leading to the production and the use of bound enzymes are three: the first one is that the enzymes can have in this way a considerable operative advantage over the free enzymes; the second one is that the chemical and physical characteristics of the enzymes can be selectively modified; the third one is that the enzymes can act as models of natural systems and enzymatic activities bound to the walls or to cellular organelles.

The various binding techniques used at present can be divided according to the following diagram which will be briefly explained:

Binding methods
Insoluble Enzymes Soluble Enzymes
Bond Entrapping Ultrafiltration Hollow
Membrane Fibers
Cross-linking Supports Gels Fibers Microcapsule
Physical Ionic Metallic Covalent
Absorption Bond Bond Bond

Cross-linking methods are base upon the covalent bonding of the molecules of the enzyme and bi- or multifunctional reactants so that threedimensional reticular aggregates completely insoluble in water are formed without using insoluble substrates. The method provides the addition of an appropriate quantity of cross-linking agent to an enzymatic solution under the most suitable conditions to form the insoluble derivative. The disadvantages of such preparation method essentially consist both in the difficulty of controlling the intermolecular cross-linking which can form large enzyme aggregates having a high activity and in that a gelatinous structure is formed which cannot assure a good mechanical resistance and then a good flow characteristic in the continuous processes.

The physical absorption of the enzyme is carried out by mixing it with the substrate under suitable conditions. Subsequently, after a convenient period of time is elapsed, the enzymatic insoluble compound is separated from the starting material by centrifugation or filtration. As no chemical reaction occurs in such method of preparation, the composition of the enzymes does not change. The main drawback of such preparation method is that the bonding forces between enzyme and substrate are generally weak so that the enzyme can be desorbed in use. As a consequence, the mean life of such preparations may be short so that they cannot be used in long-time processes.

Methods leading to the ionic bonding are mainly based upon ionic interactions between the enzymatic protein and the solid substrate having residues adapted to exchange ions. Bonds due to the ionic bonds cooperate to the formation of the resulting compounds. The main difference between such preparation methods and those based upon the physical absorption of the proteins consists in the bonding force between enzyme and substrate. As in the case of the preparation of the absorption compounds, also in the above preparation the compound is formed by contacting the substrate with the enzyme solution under softer reaction conditions than those of covalent bonding. The used substrates are generally polymers or compositions used in ion-exchange chromatography.

The complexing with transition metals is based upon the chelating properties of the transition metals, in particular titanium and zirconium. The enzymes are made insoluble by activation of molecular groups at the surface of some substrates through such metals. Besides organic substrates such as cellulose and chitin also inorganic substrates such as silica and glass wool have been used but in the latter case the results have not ever been good. Generally, such method allows high specific activities of the compounds to be held, however, the continuous activity of such compounds has a variable stability.

The covalent bonding for binding enzymes to organic and/or inorganic substrates is the most complex among the techniques leading to the bonding between the two above mentioned components as the reaction conditions for the bonding are generally harder and the manipulations to be carried out are more complicated. What is important in such preparations is that the aminoacids of the enzymes essential for the catalytic activity are not involved in the bonding with the substrate. Sometimes the protection has been obtained by the addition of the substrate or a competitive inhibitor of the enzyme activity to the reaction mixture. The easiest way for obtaining insoluble derivatives of enzymes and substrates is that the enzyme react in a solution upon the substrate already including the suitable reactants but this is only seldom possible because the substrate does not generally include them. In fact the substrate materials which are mostly used include in most cases hydroxyl groups, carboxyl groups, amino groups, and amide groups, and these groups should be activated by preliminary reactions with the aminoacid residues of the enzymes.

The entrapping methods are based upon the mixing of the enzymes with the latex of a polymer matrix or upon the storing thereof within semipermeable membranes having pores which are small enough to prevent proteins from going lost and at the same time large enough to allow substrates and products to pass through. Such methods can be applied to any enzyme having not very neutralized activities at least in comparison with the methods requiring chemical reactions.

Gel entrapping methods are based upon the enzyme entrapping within the interstitial spaces of cross-linked polymer gels insoluble in water. The polymer reticulum can be obtained by monomer, oligomer or polymer precursors by changing the solubility variables such as solvent, temperature, ionic force, and pH.

A different binding method leading to the entrapping of the enzyme into micro-cavities is the physical entrapping of an enzyme by dissolving a polymer adapted to form fibers (for example cellulose triacetate) in an organic solvent insoluble in water (for example, chloroform, methylene chloride, carbon tetrachloride) and by emulsifying such solution with the acqueous solution of the enzyme. The solution is poured in a liquid coalescent (for example, toluol or petroleum ether) which precipitates the emulsion under the form of a filament including microdrops of enzymatic solution. However, such preparation is limited to enzymes acting on substrates having a low molecular weight because of the difficulty due to the steric hindrance of the large substrates. Other problems originate from the necessity of using liquids insoluble in water as solvent of the polymer.

The enzymes can also be entrapped within microcapsules prepared along with organic polymers. The membranes enclosing the enzymes are semipermeable in comparison with substrate and products. The main disadvantages consist, however, in that such method cannot be applied to enzyme activities which catalyze reactions on substrates of high molecular weight, and that the disactivation may occur during the preparation.

The method in accordance with the present invention is different from the above mentioned methods in that it provides the absorption of biological catalytic activities of any kind onto an inert material of non-biological nature which is made of expanded clay having the following characteristics of:
- being adapted to be confined in well-delimitated regions of the space so as to form an independent solid phase which can be removed from the system, if desired;
- being porous enough as to allow the biological catalytic activities to be effectively absorbed;
- being produced in several sizes having different porosities;
- maintaining an inner complex cavity structure;
- being resistant to compressive stress;
- being adapted to stay under hydrous state for long time;
- being made of material of argillaceous origin;
- being subjected to sintering processes.

The latter aspect is essential for the preparation of the bound catalyst. In fact, even if the absorption of the biological catalytic activities is being studied for many years in the literature, especially as far as the various types of clay and the cationic substitutions on the same is concerned, the effect of any heat treatment on the absorbing property has not hitherto been considered.

From the experiments of the inventors it has been found that, in case of sintered expanded clay, the acquired chemical-physical properties allow it to be almost immediately used in the catalyst preparation process. The sintering modifies the specific surface, the bulk density, the hydration conditions, the micro- and macropores percentage, and the resistance to compressive stress, thus assuring very high absorbing capability and resistance to the desorption of the biological catalytic activities.

The type of biological catalytic activites which can be used is limited only by the availability of the same in the nature as they may also include natural cells or cells modified by biotechnological processes, natural or modified microorganisms, organelles or subcellular elements. This assures that the problem of the degradation, transformation and use of the residues, or other compositions which cannot be considered as residues, can on principle be solved independently of the nature and composition of the substrate by a further development and adaptation. This also allows a variety of materials to be used by only changing slightly the parameters of the preparation of the product which is flexible and easy to modify in accordance with the requirements.

The biological catalytic activities of interest are absorbed in the liquid phase on the sintered expanded clay after being previously dissolved in a buffer solution of suitable pH and molarity.

The obtained product can be in turn entrapped within a link of biologically inactive compositions resisting to degradation and formed by polymerization and/or copolymerization of simple or complex phenolic molecules, which is more convenient from the economic point of view and responsive to the characteristic of the natural compositions than a link formed, for example, by copolymerization of acryloamide and bis-acryloamide.

Catechol, pyrogallic acid, resorcinol or similar phenolic compositions can be used as phenolic monomers being adapted to generate radicals or ions stabilized by resonance and involving aromatic structures.

Advantageously, during the phenolic linking, biological catalytic activities can be copolymerized together with such link so that the link itself is formed by active and non-active catalytic parts which are protective against degradation.

The copolymerization of the phenol and the catalytic activity (one or more than one) can take place both by enzyme catalysis (for example by laccase, tyrosinase, peroxidase and hydrogen peroxide) adapted to promote the formation of radicals and/or ions, and by inorganic catalysis (for example by MnO₂, ammonium persulfate) and further by organic catalysis (for example by TEMED, light-activated tetramethylethyldiamina).

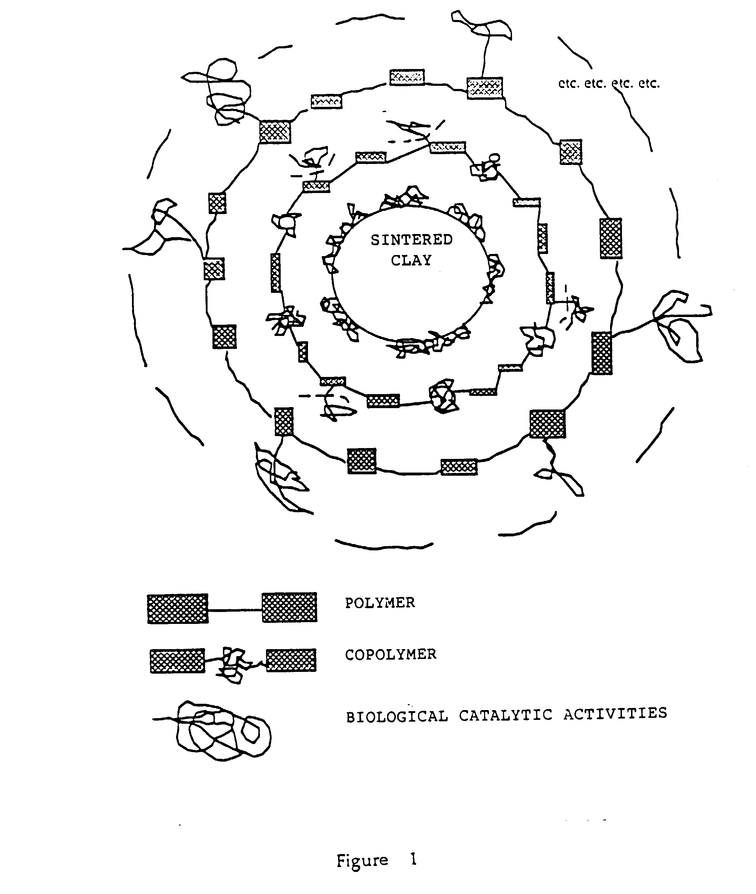

Alternately or at the same time, further enzyme activities can be linked by polyfunctional compounds to the preceding preparation so that such activities can freely fluctuate in the environment as shown in Fig. 1 illustrating a diagram of the distribution of the biological catalytic activities on the particles of sintered clay.

If necessary, a combined system formed of microorganisms and bound enzymes can be used. This chain of catalytic material of biological kind appears as a succession of layers (only one layer may be provided) surrounding the substrate, thus forming sintetically metabolic ways adapted to operate on different, simple and complex residues.

A further feature of the invention is that of catalyzing reactions having molecule of different molecular weight as substrate. In fact, the described method allows the biological catalytic activities both outside and inside the system to be bound, in the latter case the inflow of the substrates by diffusion through the links is provided and controlled in accordance to the size of the links.

From the foregoing the method of the invention provides, as claimed, in whole or in part the following steps:
a) preparing a certain quantity of sintered expanded clay;
b) preparing one or more liquid phases formed of a buffer solution having suitable pH and molarity and containing the biological catalytic activities of interest;
c) absorbing such solution on a substrate of sintered expanded clay;
d) preparing an acqueous phase containing a radical catalyst of biological kind (peroxidase and H₂O₂, laccase, tyrosinase, a.s.o.) or other kind (manganese dioxide, ammonium persulfate, TEMED, a.s.o.);
e) preparing an acqueous phase containing catechol and/or pyrogallic acid and/or resorcinol and/or similar phenolic compositions being adapted to provide radicals stabilized by resonance and involving aromatic structures;
f) adding said liquid phases to the preparation of phase c) in order to obtain a polymer and/or a copolymer of molecules of phenolic kind cross-linked to the bilogical catalytic activity at the surface of the clay particles;
alternately:
g) adding to preparation of phase a) a radical catalyst prepared as in phase d) of one or more phenolic compositions prepared as in phase e) and one or more biological catalytic activity prepared as in phase b) to provide a copolymer of one or more biological catalytic activities and phenolic compounds, which copolymer is cross-linked on sintered expanded clay.

Further features of the present invention will be more readily apparent from the following examples of the invention showing by illustrative, non-limitative way the features of the products and some uses threof.

### EXAMPLE 1

### Binding of acid phosphatase to sintered clay and preservation of its activity in the time.

The substrate formed of sintered clay (for example LECA by Laterlite) of different sizes was washed with 1N NaOH and then with distilled H₂O until the washing water gave neutral reaction. It was dried then in oven at 110°C. 10 ml of a solution of acid phosphatase (extracted from wheat germ and having the activity of 2.3 EU/mg) purchased from the Company Fluka and obtained by dissolving 0.9 mg of enzyme in a buffer of 10 ml of 0.5M acetate at pH 4.75, was added to 10 grams of said material in a flask. The total quantity of enzyme to be absorbed on to the substrate was 2.07 EU corresponding to 0.207 EU per gram of sintered clay. The reaction mixture was placed into a refrigerator at 5°C overnight. After this period of time the acid phosphatase bound to the sintered clay was washed three times with a buffer of 0.5M acetate at pH 4.75, and the washing water was collected to determine the non-bound acid phosphatase activity. The preparation was stored in a refrigerator at 5°C. Each time a test to ascertain the bound activity of the acid phosphatase had to be effected, the preparation was removed from the refrigerator, washed with a buffer of 0.5M acetate at pH 4.75, and placed into the reaction mixture. At the end of the test the preparation was washed again with a buffer of 0.5M acetate at pH 4.75 and stored into the refrigerator at 5°C till a new test. The determination of the activity of the preparation was carried out by adding 1 ml of p-nitrophenylphosphate (PNP) at the concentration of 1000 µg/ml and 4 ml of a buffer of 0.5M acetate at pH 4.75 to 10 grams of the preparation. The quantity of the product p-nitrophenol (PNF) was determined after 3 hours of reaction at 37°C by adding 1 ml of 0.5M CaCl₂ and 4 ml of 0.5N NaOH to 1 ml of the reaction mixture. After the development of the coloration the solution was passed into the spectrophotometer at 398 mµ against a white body. This was obtained by reacting 1 ml of PNP and 4 ml of a buffer of 0.5M acetate at pH 4.75 for three hours at 37°C on a quantity of sintered clay corresponding to the active catalytic preparation. 1 ml of 0.5M CaC12 and 4 ml of 0.5N NaOH was added to 1 ml of such mixture. The quantity of PNF was determined by a calibration curve.

**TABLE 1.**

| Activity of the acid phosphatase bound to sintered clay in the time. | | | |
|---|---|---|---|
| Time (days) | Residual activity (%) | Time (days) | Residual activity (%) |
| O | 87 | 119 | 59 |
| 2 | 85 | 153 | 71 |
| 8 | 100 | 253 | 61 |
| 20 | 98 | 320 | 43 |
| 35 | 80 | 389 | 33 |
| 43 | 69 | 442 | 22 |
| 55 | 61 | 504 | 25 |

In Table 1 data relative to the activity of the acid phosphatase bound to sintered clay are listed. In this table the residual activity is calculated as a percentage of the maximum measured activity, said residual activity being determined after 8 days from the preparation. The quantity of acid phosphatase bound to sintered clay corresponded to 7.3% of that contained in the buffer used for its preparation.

### EXAMPLE 2

### Influence of keeping at room temperature on the activity of the acid phosphatase bound to sintered clay.

A test with the acid phosphatase bound to sintered clay and prepared as in Example 1 was carried out by keeping the preparation over the duration of the test at room temperature instead of in the refrigerator at 5°C. All analysis and detection methods are similar to those described in Example 1.

**TABLE 2.**

| Activity of the acid phosphatase bound to sintered clay in the time. | | | |
|---|---|---|---|
| Time (days) | Residual activity (%) | Time (days) | Residual activity (%) |
| O | 82 | 153 | 45 |
| 2 | 81 | 222 | 47 |
| 8 | 100 | 254 | 33 |
| 13 | 84 | 320 | 31 |
| 20 | 80 | 389 | 24 |
| 55 | 73 | 442 | 27 |
| 119 | 43 | 504 | 22 |

In Table 2 the results of such test are listed.
The percentages are calculated on the base of the maximum activity detected after 8 days from the date of preparation.

### EXAMPLE 3

### Restoring of the acid phosphatase activity of a land partially sterilized by heating at_Ç150°C by the addition of acid phosphatase bound to sintered clay.

After having taken earth from the soil classed as sandy loam according to the FAO's nomenclature, dried in the atmosphere, sieved to provide particles of maximum size up to 2 mm, and partially sterilized by heating at 150°C for 5 hours, 5 grams were weighed to which 5 grams of the preparation of Example 1 were added. 1 ml of p-nitrophenilphosphate (PNP) dissolved in a buffer of 0.5M acetate at pH 4.75 and 10 ml of a buffer of 0.5M acetate at pH 4.75 were added to 10 grams of the mixture. After stirring for 3 hours at 37°C the reaction mixture was filtered and 1 ml thereof was taken out. 1 ml of 0.5M CaCl₂ and 4 ml of 0.5N NaOH was added to said 1 ml of the mixture. After 30 minutes the solution was read at 398 mµ. The results are shown in Table 3. In such Table there are also shown the activity of 5 grams of acid phosphatase bound to sintered clay, the activity of acid phosphatase of 5 grams of the soil, and the activity of the acid phosphatase of 5 grams of the partially sterilized soil. The acid phosphatase activity of the partially sterilized soil was restored by the bound activity by about 40%.

**TABLE 3.**

| Acid phosphatase activity of the soil, activity of the enzyme bound to sintered clay, and activity of the mixture thereof. | |
|---|---|
| Preparation | Activity (optical density at 398mµ) |
| Non-sterilized land | 0.643 |
| Partially sterilized land | 0.277 |
| Bound phosphatase | 0.258 |
| Bound phosphatase + partially sterilized land | 0.571 |

### EXAMPLE 4

### Absorption of the β-glucosidase on to sintered clay and preservation of its activity in the time.

The substrate formed of sintered clay of several sizes was washed with 1N NaOH and distilled H₂O until the washing water gave neutral reaction, then it was dried in oven at 110°C. 100 ml of a solution of β-glucosidase (extracted from sweet almond paste and having the activity of 4.4 EU/mg) purchased from the Company BDH and obtained by dissolving 50 mg of enzyme in 250 ml of a buffer of 0.1M acetate at pH 5.0, was added to 100 grams of the above material in a flask. The total quantity of enzyme to be absorbed on the substrate was 8.8 EU corresponding to 0.88 EU/gram of sintered clay. The reaction mixture was reacted in a refrigerator at 5°C overnight.
After this period of time the β-glucosidase bound to sintered clay was washed with a buffer of 0.1M acetate at pH 5.0, and the washing water was collected in order to determine the non-absorbed β-gluosidase activity. The preparation was stored in a refrigerator at 5°C. Each time a test to ascertain the bound β-glucosidase activity had to be effected, the preparation was removed from the refrigerator, washed with a buffer of 0.5M of acetate at pH 5.0, and placed into the reaction mixture. At the end of the test the preparation was washed again with a buffer of 0.1M acetate at pH 5.0 and placed into the refrigerator at 5°C till a new test. The determination of the activity of the preparation was carried out by adding 4 ml of salicin at the concentration of 1% in a buffer of 0.1M acetate at pH 5.0 to 100 milligrams of the preparation. After 1 hour incubation at 37°C the reaction mixture was separated from the solid phase and brought for 5 minutes at 100°C, then placed in ice until the room temperature was reached. After such period of time 0.1 ml was taken and analyzed for the reducing sugars according to the method of Nelson and Somogyi by a readout with a spectrophotometer at 520 mµ against a white body. This was obtained by contacting at 37°C for 1 hour 4 ml of salicin at the concentration of 1% in a buffer of 0.1M acetate at pH 5.0 with a quantity of sintered clay corresponding to the activated enzyme preparation, and following the same procedure as before. The quantity of reducing sugars were calculated by a calibration curve drawn each time.

**TABLE 4.**

| Activity of the β-glucosidase bound to sintered clay in the time. | | | |
|---|---|---|---|
| Time (days) | Residual activity (%) | Time (days) | Residual activity (%) |
| O | 86 | 49 | 94 |
| 2 | 89 | 84 | 95 |
| 8 | 87 | 102 | 69 |
| 18 | 100 | 138 | 71 |
| 35 | 91 | 194 | 36 |

In Table 4 data relative to the activity of the β-qlucosidase bound to sintered clay are listed assuming as a maximum the activity measured after 18 days from the beginning of the test. The quantity of glucosidase bound to sintered clay corresponds to 16.2% of that contained in the buffer used for the preparation.

### EXAMPLE 5

### Activity of the β-glucosidase bound to sintered clay after storing at room temperature for some time.

One hundred grams of β-glucosidase bound to sintered clay was prepared as described in Example 4.

Such preparation was stored at room temperature and 10 grams thereof was taken at predetermined time in order to carry out the test of the duration of the activity measured as in Example 4. In Table 5 the results of the test are listed.

**TABLE 5.**

| Activity of the β-glucosidase bound to sintered clay and stored at room temperature for different times. | | | | | |
|---|---|---|---|---|---|
| Storage time (days) | | | | | |
| 16 | | 26 | | 43 | |
| Time (days) | Activity (%) | Time (days) | Activity (%) | Time (days) | Activity (%) |
| O | 85 | 0 | 100 | 0 | 100 |
| 10 | 100 | 17 | 86 | 14 | 81 |
| 27 | 94 | 31 | 95 | 49 | 54 |
| 41 | 99 | 66 | 96 | 67 | 70 |
| 76 | 100 | 84 | 86 | 103 | 51 |
| 94 | 80 | 120 | 79 | 159 | 49 |
| 130 | 92 | 176 | 47 | --- | --- |
| 186 | 64 | --- | --- | --- | --- |

### EXAMPLE 6

### Restoring of the β-glucosidase activity of a land sterilized by heating at 150°C by means of the addition of β-glucosidase bound to sintered clay.

After having taken earth from the soil classed as sandy loam according to the FAO's nomenclature, dried in the atmosphere, sieved to provide particles of maximum size up to 2 mm, and sterilized by heating at 150°C for 48 hours, 5 grams were weighed to which 5 grams of the preparation of Example 4 were added. 4 ml of salicin at the concentration of 1% in a buffer of 0.1M acetate at pH 5.0 and 6 ml of a buffer of 0.5M acetate at pH 5.0 were added to 10 grams of the mixture. After stirring for 1 hour at 37°C the reaction mixture was filtered and 0.1 ml thereof was taken out. The reducing sugars were determined on the above quantity as in Example 4. The results are shown in Table 6. In such Table there are also shown the activity of the β-glucosidase bound to 5 grams of sintered clay of the tested soil. The activity of the β-glucosidase of the soil was increased by 6 times due to the addition of the insoluble preparation.

**TABLE 3.**

| β-glucosidase activity of the soil, activity of the enzyme bound to sintered clay, and activity of the mixture thereof. | |
|---|---|
| Preparation | Activity (optical density at 398 mµ) |
| Non-sterilized land | 0.062 |
| Partially sterilized land | 0.005 |
| Bound β-glucosidase | 0.297 |
| Bound β-glucosidase + partially sterilized land | 0.342 |

### EXAMPLE 7

### Absorption of cellulase onto sintered clay and preservation of its activity in the time.

The substrate formed of sintered clay of several sizes was washed with 1N NaOH and distilled H₂O until the washing water gave neutral reaction. Finally it was dried in oven at 110°C. 100 ml of a solution of cellulase (extracted from Tricoderma viride and having the activity of 0.02 EU/mg) purchased from the Company BDH and obtained by dissolving 100 mg of enzyme in 200 ml of a buffer of 0.1M acetate at pH 5.0, was added to 100 grams of the above material in a flask. The total quantity of enzyme to be absorbed onto the substrate was 2.0 EU corresponding to 0.02 EU/gram of sintered clay. The reaction mixture was reacted in a refrigerator at 5°C overnight. After this period of time the cellulase bound to sintered clay was washed three times with a buffer of 0.1M acetate at pH 5.0, and the washing water was collected in order to determine the non-absorbed cellulase activity. The preparation was stored in a refrigerator at 5°C. Each time a test to ascertain the activity of the bound cellulase had to be effected, the preparation was removed from the refrigerator, washed with a buffer of 0.1M of acetate ad pH 5.0, and placed into the reaction mixture. At the end of the test the preparation was washed again with a buffer of 0.1M acetate at pH 5.0 and stored into the refrigerator at 5°C till a new test. The determination of the activity of the preparation was carried out by adding 5 ml of carboxymethil cellulose (CMC) at the concentration of 1% in a buffer of 0.1M acetate at pH 5.0, and 3 ml of a buffer of 0.1M acetate at pH 5.0 to 10 grams of the preparation. After 1 hour incubation at 37°C the reaction mixture was separated from the solid phase and added to 1 ml of 0.5M NaOH. Then 1 ml was taken and analyzed for the reducing sugars according to the method of Nelson and Somogyi by a readout with a spectrophotometer at 520 mµ against a white body. This was obtained by contacting at 37°C for 1 hour 5 ml of CMC at the concentration of 1% in a buffer of 0.1M acetate at pH 5.0 and 3 ml of a buffer of 0.1M acetate at pH 5.0 with a quantity of sintered clay corresponding to the activated enzyme preparation, and following the same procedure as before. The quantity of reducing sugars was calculated by a calibration curve drawn each time.

**TABLE 7.**

| Activity of the cellulase bound to sintered clay in the time. | |
|---|---|
| Time (days) | Residual activity (%) |
| O | 100 |
| 22 | 47 |
| 38 | 30 |
| 68 | 21 |
| 125 | 11 |

The activity of the bound cellulase corresponds to 5.25% of that contained in the enzyme solution used for the preparation of the bound cellulase.

### EXAMPLE 8

### Activity of the cellulase bound to sintered clay after storing at room temperature for some time.

One hundred grams of cellulase bound to sintered clay was prepared as described in Example 7. Such preparation was stored at room temperature and 10 grams thereof was taken out at predetermined time in order to carry out the test of the duration of the activity by comparing the latter with the activity of the cellulase bound to freshly prepared sintered clay as described in Example 7. In Table 8 the results of the test are listed.

**TABLE 8.**

| Activity of the cellulase bound to sintered clay and stored at room temperature for different times. | | | |
|---|---|---|---|
| Storing time (days) | | | |
| 22 | | 38 | |
| Time (days) | Activity (%) | Time (days) | Activity (%) |
| 0 | 100 | 0 | 100 |
| 16 | 42 | 14 | 40 |
| 30 | 26 | 51 | 16 |
| 67 | 9 | --- | --- |

### EXAMPLE 9

### Preparation and enzyme activity of a copolymer formed of pyrogallic acid and acid phosphatase by peroxidase and H₂O₂ and cross-linked on β-gluocosidase absorbed onto sintered expanded clay.

Fifty grams of β-glucosidase bound to sintered clay were prepared as described in Example 4. 7.5 ml of a solution containing pyrogallic acid (25 mg/100 ml), 10 ml of a solution containing peroxidase (25 mg/250 ml) and 2.5 ml of 0.06% H₂O₂ were added to 5 grams of the above preparation. Then 5 ml of a solution of acid phosphatase (18 mg/200 ml of a buffer of 0.1M acetate at pH 5.0) was added to such preparation. After slow stirring for 2 hours the preparation was filtered and washed with distilled water, and then stored in refrigerator at 5°C. Both glucosidase and phosphatase activities of such preparation were measured at different time intervals, at the end of each of them the preparation was washed with distilled water and stored in refrigerator at 5°C till the new test. The results of such tests performed as described in Examples 1 and 4 are listed in Table 9.

**Table 9.**

| Activity of β-glucosidase bound to sintered clay in the time, and activity of acid phosphatase copolymerized with pyrocatechol by peroxidase and H₂O₂. | | |
|---|---|---|
| Time | β-glucosidase | Acid Phosphatase |
| (days) | residual activity (%) | |
| 0 | 87 | 75 |
| 2 | 100 | 100 |
| 8 | 85 | 78 |

### EXAMPLE 10

### Preparation and enzyme activity of a copolymer formed of pyrogallic acid and acid phosphatase by ammonium persulfate and cross-linked on β-gluocosidase absorbed onto sintered expanded clay.

Fifty grams of β-glucosidase bound to sintered clay were prepared as described in Example 5. 0.5 ml of a solution containing pyrogallic acid (25 mg/100 ml) and 10 ml of a solution containing persulfate (100 mg/100 ml) were added to 5 grams of the above preparation. Then 5 ml of a solution of acid phosphatase (18 mg/200 ml of a buffer of 0.1M acetate at pH 5.0) was added to such preparation.

After slow stirring for 1 hour the preparation was filtered and washed with distilled water, and then stored in refrigerator at 5°C. Both β-glucosidase and acid phosphatase activities of such preparation were measured at different time intervals, at the end of each of them the preparation was washed with distilled water and stored in refrigerator at 5°C till the new test. The results of such tests performed as described in Examples 1 and 4 are listed in Table 10.

**Table 10.**

| Activity of β-glucosidase bound to sintered clay in the time, and activity of acid phosphatase copolymerized with pyrocatechol byperoxidase and H₂O₂. | | |
|---|---|---|
| Time | β-glucosidase | Acid Phosphatase |
| (days) | residual activity (%) | |
| 0 | 83 | 78 |
| 2 | 100 | 100 |
| 8 | 85 | 64 |
| 9 | 88 | 71 |

### EXAMPLE 11

### Preparation and enzyme activity of a copolymer formed of catechol and β-glucosidase by manganese dioxide and cross-linked on sintered expanded clay.

A solution consisting of 1 ml of a solution of MnO2 (100 mg/ml), 1 ml of a solution of catechol (250 mg/100 ml), and 5 ml of a solution of β-glucosidase obtained by dissolving 50 mg of enzyme in 250 ml of a buffer of 0.1M acetate at pH 5.0 was added to 5 grams of sintered expanded clay.

After slow stirring for 1 hour the preparation was filtered and washed with distilled water, and then stored in refrigerator at 5°C. The β-glucosidase activity of such preparation was measured at different time intervals, at the end of each of them the preparation was washed with distilled water and stored in refrigerator at 5°C till the new test. The results of such tests performed as described in Example 4 are listed in Table 11.

**Table 11.**

| Activity of β-glucosidase copolymerized with catechol by MnO2 on sintered expanded clay in the time. | |
|---|---|
| Time | β-glucosidase |
| (days) | residual activity (%) |
| 0 | 98 |
| 2 | 100 |
| 12 | 87 |
| 27 | 81 |

### EXAMPLE 12

### Preparation and enzyme activity of a copolymer formed of pyrogallic acid and β-glucosidase by TEMED, cross-linked on sintered expanded clay.

A solution consisting of 30 ul of a solution of TEMED (80 mg/100 ml), 1 ml of a solution of pyrogallic acid (250 mg/100 ml), and 5 ml of a solution of β-glucosidase obtained by dissolving 50 mg of enzyme in 250 ml of a buffer of 0.1M acetate at pH 5.0 was added to 5 grams of sintered expanded clay.

After slow stirring for 1 hour the preparation was filtered and washed with distilled water, and then stored in refrigerator at 5°C. The β-glucosidase activity of such preparation was measured at different time intervals, at the end of each of them the preparation was washed with distilled water and stored in refrigerator at 5°C till the new test. The results of such tests performed as described in Example 4 are listed in Table 12.

**Table 12.**

| Activity of β-glucosidase copolymerized with pyrogallic acid by TEMED on sintered expanded clay in the time. | |
|---|---|
| Time | β-glucosidase |
| (days) | residual activity (%) |
| 0 | 100 |
| 2 | 85 |
| 12 | 63 |
| 27 | 48 |

## Claims

1. A method of binding biological catalytic activities to a solid substrate, characterized by the fact that it comprises a first step of:
- absorbing one or more biological catalytic activities onto the surface of a solid substrate made of sintered expanded clay; and a second step of:
- polymerizing and/or copolymerizing simple or complex molecules of phenolic kind on the surface of said treated solid substrate, said molecules providing radicals or ions stabilized by resonance and involving aromatic structures, so that said biological catalytic activities are entrapped within a link of biologically inactive compositions resistant to degradation.

2. The method of claim 1, characterized in that the second step consists in the copolymerizing at the surface of said treated solid substrate one or more biological catalytic activities, which are alike or different from the preceding activities previously absorbed onto the surface of the same solid substrate made of sintered expanded clay, with phenolic compound acting as monomers in polymerization or copolymerization reactions of radical or ionic kind.

3. A method of binding biological catalytic activities to a solid substrate, characterized by the step of copolymerizing the biological catalytic activities of interest with phenolic compounds acting as monomers in polymerization or copolymerization reactions of radical or ionic kind on a solid substrate of sintered expanded clay so as to obtain a phenolic link formed of activated and non-activated catalytic parts protecting against degradation.

4. The method of the preceding claims, characterized in that the sintered expanded clay has a porosity of 25% to 75%.

5. The method of claims 1 to 3, characterized in that the weight ratio between phenolic monomer and units of biological catalytic activities ranges from 1:0.01 to 1:250.

6. The method of claims 1 to 3, characterized in that the copolymer phenol-biological catalytic activity (one or more than one) is formed through enzyme catalysis adapted to form radicals or ions.

7. The method of claim 6, wherein laccase, tyrosinase, peroxidase and hydrogen peroxide are used in the enzyme catalysis.

8. The method of claims 1 to 3, characterized in that the copolymer phenol-biological catalytic activity (one or more than one) is formed through inorganic catalysis by adding the catalyst so as to provide a varying proportion from 1:1:0.01 to 1:100:2500 among weight in grams of inorganic compound, weight in grams of phenolic compound and units of biological catalytic activity.

9. The method of claim 8, characterized in that the catalyst is ammonium persulfate.

10. The method of claim 8, characterized in that the catalyst is manganese dioxide.

11. The method of claims 1 to 3, characterized in that the copolymer phenol-biological catalytic activity (one or more than one) is formed through organic catalysis by adding the catalyst so as to provide a varying proportion from 0.1:1:0,01 to 0,1:100:2500 among weight in grams of organic compound, weight in grams of phenolic compound and units of biological catalytic activity.

12. The method of claim 11, characterized in that the organic catalyst is TEMED.

13. A substrate for transformation reaction of compositions of various kind, characterized in that it is obtained by the method of claim 1.

14. A substrate for transformation reaction of compositions of various kind, characterized in that it is obtained by the method of claim 2.

15. A substrate for transformation reaction of compositions of various kind, characterized in that it is obtained by the method of claim 3.

16. Use of the substrate, as claimed in any one of claims 13 to 15, as catalyst in processes to modify and improve the efficiency of not very fertile lands to which residual or restoring products are added.

17. Use of the substrate, as claimed in any one of claims 13 to 15, as catalyst in decontamination processes in the agriculture and in the forest and environmental fields.

18. Use of the substrate, as claimed in any one of claims 13 to 15, as catalyst in decontamination processes in the civil field, for example, in the city dumps and on the occasion of accidental poisonous, noxious leakages.

19. Use of the substrate, as claimed in any one of claims 13 to 15, as catalyst in decontamination processes in the industrial field, for example tank spillage, or in processes to reduce the pollution by cleaning tanks and the like.

20. Use of the substrate, as claimed in any one of claims 13 to 15, as catalyst in reactions for the depuration of sludges and/or waste water from industrial plants.

21. Use of the substrate, as claimed in any one of claims 13 to 15, as catalyst in processes of depuration of mineral and non-mineral fuel through the degradation of the contaminants.

22. Use of the substrate, as claimed in any one of claims 13 to 15, as catalyst in processes of depuration of water intended for civil and industrial use through the building of suitable beds or devices.

23. Use of the substrate, as claimed in any one of claims 13 to 15, as catalyst in processes of preparation and depuration of compositions in the industrial field.

24. Use of the substrate, as claimed in any one of claims 13 to 15, as catalyst in processes of depuration of oil compositions in earth, river, lake and sea environments.

## Patentansprüche

1. Verfahren zur Bindung biologischer katalytischer Aktivitäten an einen Festträger,
dadurch gekennzeichnet,
daß es folgenden ersten Schritt aufweist:
- Absorbieren einer oder mehrerer biologischer katalytischer Aktivitäten auf der Oberfläche eines aus gesintertem expandierten Ton hergestellten Festträgers; und als zweiten Schritt:
- Polymerisieren und/oder Copolymerisieren einfacher oder komplexer Moleküle vom Phenoltyp auf die Oberfläche des behandelten Festträgers, wobei die Moleküle Radikale oder Ionen darstellen, die durch Resonanz stabilisiert sind und aromatische Strukturen aufweisen, so daß die biologischen katalytischen Aktivitäten in einer gegen Abbau beständigen Verknüpfung aus biologisch inaktiven Zusammensetzungen eingeschlossen sind.

2. Verfahren nach Anspruch 1,
dadurch gekennzeichnet,
daß der zweite Schritt in der Copolymerisierung einer oder mehrerer biologischer katalytischer Aktivitäten an die Oberfläche des behandelten Festträgers besteht, wobei sich diese Aktivitäten von den vorher auf die Oberfläche des gleichen Festträgers aus gesintertem expandierten Ton absorbierten vorherigen Aktivitäten unterscheiden oder ihnen gleichen, wobei die Phenolverbindungen als Monomere in Polymerisations- oder Copolymerisationsreaktionen vom Radikaltyp oder Ionentyp wirken.

3. Verfahren zur Bindung biologischer katalytischer Aktivitäten an einen Festträger, gekennzeichnet durch das Copolymerisieren der interessierenden biologischen katalytischen Aktivitäten mit Phenolverbindungen, die als Monomere in Polymerisations- oder Copolymerisationsreaktionen vom Radikal- oder Ionentyp an einen Festträger aus gesintertem expandierten Ton wirken, um eine aus aktivierten oder nicht aktivierten katalytischen Teilen gebildete Phenolverknüpfung zu erhalten, die gegen Abbau schützt.

4. Verfahren nach einem der vorhergehenden Ansprüche,
dadurch gekennzeichnet,
daß der gesinterte expandierte Ton eine Porosität von 25% bis 75% aufweist.

5. Verfahren nach einem der Ansprüche 1 bis 3,
dadurch gekennzeichnet,
daß das Gewichtsverhältnis zwischen dem Phenolmonomer und den Einheiten der biologischen katalytischen Aktivitäten im Bereich von 1:0,01 bis 1:250 liegt.

6. Verfahren nach einem der Ansprüche 1 bis 3,
dadurch gekennzeichnet,
daß die Copolymer-Phenol-biologische katalytische Aktivität (eine oder mehr als eine) durch Enzymkatalyse gebildet wird, die zur Bildung von Radikalen oder Ionen ausgelegt ist.

7. Verfahren nach Anspruch 6, wobei Laccase, Tyrosinase, Peroxidase und Wasserstoffperoxid bei der Enzymkatalyse verwendet werden.

8. Verfahren nach einem der Ansprüche 1 bis 3,
dadurch gekennzeichnet,
daß die Copolymer-Phenol-biologische katalytische Aktivität (einer oder mehr als eine) durch anorganische Katalyse durch Zugabe des Katalysators gebildet wird, um einen unterschiedlichen Anteil von 1:1:0,01 bis 1:100:2500, ausgedrückt in Grammgewicht der anorganischen Verbindung, Grammgewicht der Phenolverbindung und Einheiten der biologischen katalytischen Aktivität, zu erhalten.

9. Verfahren nach Anspruch 8,
dadurch gekennzeichnet,
daß der Katalysator Ammoniumpersulfat ist.

10. Verfahren nach Anspruch 8,
dadurch gekennzeichnet,
daß der Katalysator Mangandioxid ist.

11. Verfahren nach einem der Ansprüche 1 bis 3,
dadurch gekennzeichnet,
daß die Copolymer-Phenol-biologische katalytische Aktivität (eine oder mehr als eine) durch organische Katalyse durch Zugabe des Katalysators gebildet wird, um einen unterschiedlichen Anteil von 0,1:1:0,01 bis 0,1:100:2500, ausgedrückt in Grammgewicht der organischen Verbindung, Grammgewicht der Phenolverbindung und Einheiten der biologischen katalytischen Aktivität, zu erhalten.

12. Verfahren nach Anspruch 11,
dadurch gekennzeichnet,
daß der organische Katalysator TEMED ist.

13. Träger für die Transformationsreaktion der unterschiedlichen Zusammensetzungen,
dadurch gekennzeichnet,
daß er durch das Verfahren nach Anspruch 1 erhalten wird.

14. Träger für die Transformationsreaktion der unterschiedlichen Zusammensetzungen,
dadurch gekennzeichnet,
daß er durch das Verfahren nach Anspruch 2 erhalten wird.

15. Träger für die Transformationsreaktion der unterschiedlichen Zusammensetzungen,
dadurch gekennzeichnet,
daß er durch das Verfahren nach Anspruch 3 erhalten wird.

16. Verwendung des Trägers nach einem der Ansprüche 13 bis 15 als Katalysator in Verfahren zur Modifizierung und Verbesserung der Effizienz von nicht sehr fruchtbarem Boden, zu dem Abfall- oder Wiederherstellungsprodukte zugegeben werden.

17. Verwendung des Trägers nach einem der Ansprüche 13 bis 15 als Katalysator bei Dekontaminationsverfahren in der Landwirtschaft und auf dem Forst- und Umweltgebiet.

18. Verwendung des Trägers nach einem der Ansprüche 13 bis 15 als Katalysator bei Dekontaminierungsprozessen auf zivilem Gebiet, beispielsweise bei Stadtmüll und bei Unfällen, bei denen giftige, schädliche Ausflüsse auftreten.

19. Verwendung des Trägers nach einem der Ansprüche 13 bis 15 als Katalysator in Dekontaminierungsprozessen auf industriellem Gebiet, beispielsweise bei Tanklecks oder bei Prozessen zur Verringerung der Verschmutzung durch Reinigungstanks und ähnliche Prozesse.

20. Verwendung des Trägers nach einem der Ansprüche 13 bis 15 als Katalysator bei Reaktionen zur Reinigung von Schlämmen und/oder Abwasser aus Industrieanlagen.

21. Verwendung des Trägers nach einem der Ansprüche 13 bis 15 als Katalysator bei Prozessen zur Reinigung von Mineralund Nicht-Mineralöl durch Abbau der Kontaminanten.

22. Verwendung des Trägers nach einem der Ansprüche 13 bis 15 als Katalysator in Prozessen zur Reinigung von Brauchund Industriewasser durch die Bildung geeigneter Bette oder Vorrichtungen.

23. Verwendung des Trägers nach einem der Ansprüche 13 bis 15 als Katalysator in Prozessen zur Herstellung und Reinigung von Zusammensetzungen auf industriellem Gebiet.

24. Verwendung des Trägers nach einem der Ansprüche 13 bis 15 als Katalysator in Prozessen zur Reinigung von Ölzusammensetzungen in Erd-, Fluß-, See- und Meerumgebung.

## Revendications

1. Procédé de liaison d'activités catalytiques biologiques à un substrat solide, caractérisé par le fait qu'il comprend une première étape consistant à:
- absorber une ou plusieurs activités catalytiques biologiques sur la surface d'un substrat solide fait d'argile expansée frittée; et une seconde étape consistant à:
- polymériser et/ou copolymériser des molécules simples ou complexes de type phénolique sur la surface dudit substrat solide traité, lesdites molécules fournissant des radicaux ou des ions stabilisés par résonance et mettant en jeu des structures aromatiques, de manière que lesdites activités catalytiques biologiques soient piégées au sein d'un enchaînement de compositions inactives sur le plan biologique, résistantes à la décomposition.

2. Procédé selon la revendication 1, caractérisé en ce que la seconde étape se compose d'une copolymérisation, sur la surface dudit substrat solide traité, d'une ou de plusieurs activités biologiques, qui sont semblables aux précédentes activités absorbées préalablement sur la surface de ce substrat solide fait d'argile expansée frittée, ou qui en diffèrent, le composé phénolique jouant le rôle de monomères dans les réactions de polymérisation ou de copolymérisation de type radicalaire ou ionique.

3. Procédé de liaison d'activités catalytiques biologiques à un substrat solide, caractérisé par l'étape consistant à copolymériser les activités catalytiques biologiques présentant un intérêt avec des composés phénoliques jouant le rôle de monomères dans les réactions de polymérisation ou de copolymérisation de type radicalaire ou ionique, sur un substrat solide d'argile expansée frittée, de manière à obtenir un enchaînement phénolique formé de parties catalytiques activées et non activées, protégées de la décomposition.

4. Procédé selon les revendications précédentes, caractérisé en ce que l'argile expansée frittée possède une porosité de 25% à 75%.

5. Procédé selon les revendications 1 à 3, caractérisé en ce que le rapport pondéral entre le monomère phénolique et les motifs d'activités catalytiques biologiques s'échelonne de 1:0,01 à 1:250.

6. Procédé selon les revendications 1 à 3, caractérisé en ce que le copolymère phénol/activité catalytique biologique (une ou plusieurs) est formé par catalyse enzymatique adaptée à la formation de radicaux ou d'ions.

7. Procédé selon la revendication 6, dans lequel on utilise de la laccase, de la tyrosinase, de la peroxydase et du peroxyde d'hydrogène dans la catalyse enzymatique.

8. Procédé selon les revendications 1 à 3, caractérisé en ce que le copolymère phénol/activité catalytique biologique (une ou plusieurs) est formé par catalyse minérale, par addition du catalyseur de manière à obtenir une proportion variable de 1:1:0,01 à 1:100:2500, exprimée en poids en grammes de composé minéral, poids en grammes de composé phénolique et motifs d'activité catalytique biologique.

9. Procédé selon la revendication 8, caractérisé en ce que le catalyseur est le persulfate d'ammonium.

10. Procédé selon la revendication 8, caractérisé en ce que le catalyseur est le dioxyde de manganèse.

11. Procédé selon les revendications 1 à 3, caractérisé en ce que le copolymère phénol/activité catalytique biologique (une ou plusieurs) est formé par catalyse organique, par addition du catalyseur de manière à obtenir une proportion variable de 0,1:1:0,01 à 0,1:100:2500, exprimée en poids en grammes de composé organique, poids en grammes de composé phénolique et motifs d'activité catalytique biologique.

12. Procédé selon la revendication 11, caractérisé en ce que le catalyseur organique est la TEMED.

13. Substrat pour la réaction de transformation de compositions de différents types, caractérisé en ce qu'il est obtenu par le procédé de la revendication 1.

14. Substrat pour la réaction de transformation de compositions de différents types, caractérisé en ce qu'il est obtenu par le procédé de la revendication 2.

15. Substrat pour la réaction de transformation de compositions de différents types, caractérisé en ce qu'il est obtenu par le procédé de la revendication 3.

16. Utilisation du substrat, selon l'une quelconque des revendications 13 à 15, comme catalyseur dans des procédés servant à modifier et à améliorer l'efficacité de terres pas très fertiles auxquelles sont ajoutés des produits résiduels ou reconstituants.

17. Utilisation du substrat, selon l'une quelconque des revendications 13 à 15, comme catalyseur dans des procédés de décontamination dans les domaines de l'agriculture et des forêts et le domaine de l'environnement.

18. Utilisation du substrat, selon l'une quelconque des revendications 13 à 15, comme catalyseur dans des procédés de décontamination dans le domaine civil, par exemple les décharges publiques et à l'occasion de fuites accidentelles nocives provoquant des empoisonnements.

19. Utilisation du substrat, selon l'une quelconque des revendications 13 à 15, comme catalyseur dans des procédés de décontamination dans le domaine industriel, par exemple les fuites de cuves, ou dans les procédés servant à réduire la pollution par le nettoyage de cuves et analogues.

20. Utilisation du substrat, selon l'une quelconque des revendications 13 à 15, comme catalyseur dans des réactions de dépuration de boues et/ou d'eaux usées provenant d'installations industrielles.

21. Utilisation du substrat, selon l'une quelconque des revendications 13 à 15, comme catalyseur dans des procédés de dépuration de combustibles minéraux et non minéraux par décomposition des contaminants.

22. Utilisation du substrat, selon l'une quelconque des revendications 13 à 15, comme catalyseur dans des procédés de dépuration d'eau destinée à l'usage civil et industriel par construction de lits ou de dispositifs convenables.

23. Utilisation du substrat, selon l'une quelconque des revendications 13 à 15, comme catalyseur dans des procédés de préparation et de dépuration de compositions dans le domaine industriel.

24. Utilisation du substrat, selon l'une quelconque des revendications 13 à 15, comme catalyseur dans des procédés de dépuration de compositions huileuses dans les environnements du sol, des rivières, des lacs et des mers.
